(19) Europäisches Patentamt
European Patent Office
Office européen des brevets

(11) Veröffentlichungsnummer: **0 173 197**
**B1**

(12) # EUROPÄISCHE PATENTSCHRIFT

(45) Veröffentlichungstag der Patentschrift: **01.07.87**

(51) Int. Cl.⁴: **C 07 C 149/14,** C 08 F 20/38

(21) Anmeldenummer: **85110336.6**

(22) Anmeldetag: **19.08.85**

(54) **Ungesättigte Diester von Thia-Alkandiolen.**

(30) Priorität: **30.08.84 DE 3431844**

(43) Veröffentlichungstag der Anmeldung:
**05.03.86 Patentblatt 86/10**

(45) Bekanntmachung des Hinweises auf die Patenterteilung:
**01.07.87 Patentblatt 87/27**

(84) Benannte Vertragsstaaten:
**DE FR GB**

(56) Entgegenhaltungen:
**CH-A-100 181**
**CH-A-406 193**
**US-A-4 340 707**

(73) Patentinhaber: **Röhm GmbH, Kirschenallee Postfach 4242, D-6100 Darmstadt 1 (DE)**

(72) Erfinder: **Hohage, Heinz- Jürgen, Dr., In der Wildnis 3, D-6109 Mühltal (DE)**

**EP 0 173 197 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

## Beschreibung

### Gebiet der Erfindung

Die Erfindung betrifft ungesättigte Diester von Thia-Alkandiolen, die in radikalischer Reaktion polymerisiert werden können. Die Verbindungen eignen sich insbesondere zur Vernetzung von Polymerketten.

### Stand der Technik

Der Technik stehen eine große Anzahl radikalisch polymerisierbarer Monomerer zur Verfügung.

Technisch bedeutend sind u.a. die Ester der Acryl- und der Methacrylsäure. Auf der Grundlage speziell des Methylmethacrylats wurde eine Klasse hochwertiger Kunststoffe entwickelt, die im vielen Bereichen der Technik ausgedehnte Anwendung findet. (Vgl. R. Vieweg, F. Esser in Kunststoff-Handbuch, Band IX, "Polymethacrylate", C. Hanser Verlag, 1975).

Die Eigenschaften der Kunststoffe können außer durch die Wahl der Monomeren und über das Molekulargewicht auch durch Vernetzung beeinflußt werden. Unter Vernetzung wird die räumliche Verknüpfung von Polymerketten untereinander zu einem Netzwerk verstenden.

Eine "gezielte" Vernetzung kann man durch Copolymerisation mit polyvalenten (Meth)acrylverbindungen erreichen. Solche Monomeren enthalten mindestens zwei Vinyldoppelbindungen im Molekül. (Vgl. H. Rauch-Puntigam, Th. Völker im "Acryl- und Methacrylverbindungen, Springer Verlag 1967).

Angewendet werden z. B. die Acryl- bzw. Methacrylsäureester von Di- und Polyolen, die Di- und Polyamide der entsprechenden Di- und Polyamine wie z. B. Glykoldi(meth)acrylat, Methylenbis(meth)acrylamid, trivalente Vernetzungsmittel wie Trimethylolpropantrimethacrylat, aber auch Monomere mit mehreren verschiedenartigen polymerisationsfähigen Gruppen im Molekül wie das Allylacrylat und -methacrylat.

### Aufgabe und Lösung

Die vielfältigen Probleme und wechselnden Aufgabenstellungen bei der gezielten Polymersynthese machen die Entwicklung neuer, den Polymerisationsverlauf und die Polymerisationsprodukte beeinflussender Mittel wünschenswert.

Es wurde nun gefunden, daß umgesättigte Diester von Thia-Alkandiolen der Formel I

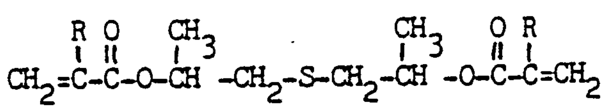

I

worin R für Wasserstoff oder Methyl steht, die Forderungen der Technik in besonderem Maße erfüllen.

Die neuen Diester der Formel I können in an sich bekannter Weise z. B. durch Umsetzung von mindestens zwei Molen eines aktivierten Derivats der Acryl- bzw. der Methacrylsäure der Formel II

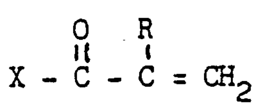

II

worin X für Chlor oder Brom oder für einen Rest

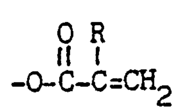

worin R die oben angegebene Bedeutung besitzt, mit Thiodipropylenglykol, gegebenenfalls in Anwesenheit eines Säureakzeptors (vgl. US-PS 1 951 782) oder durch Veresterung der (Meth)acrylsäure mit

Thiodipropylenglykol, vorzugsweise unter Binden des gebildeten Wassers mittels geeigneter dehydratisierender Agentien oder vorzugsweise durch Umesteung der niederen Ester der Acryl- bzw. der Methacrylsäure der Formel III

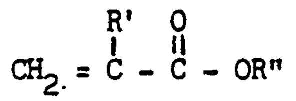

III

worin R' für Methyl oder Wasserstoff und R'' für Methyl, Ethyl oder Propyl oder Butyl steht, mit Thiodipropylenglykol, vorzugsweise in Gegenwart von Umesterungskatalysatoren hergestellt werden.

(Vgl. H. Rauch-Puntigam, Th. Völker "Acryl- und Methacrylverbindungen", pp. 36-40, US-PS 2 138 763).

Als Umesterungskatalysatoren kommen z.B. basische Katalysatoren wie Alkalialkoholate (vgl. CH-PS 239 750), saure Katalysatoren wie p-Toluolsulfonsäure, Schwefelsäure (GB-PS 461 979, GB-PS 960 005) oder vorzugsweise Orthotitansäureester (GB-PS 960 005 und GB-PS 962 928) in Frage. Zweckmäßigerweise werden die Umsetzungen in Gegenwart von an sich bekannten Polymerisationsinhibitoren wie Chinonen, Benzthiazin, Phenolen, Methylenblau, aromatischen Aminen (Diphenylamin) oder Nitroverbindungen gegebenenfalls auch Kupfer- oder Eisensalzen durchgeführt.

Die anzuwendenden Inhibitormengen liegen meist in der Größenordnung von 0,01 bis 0.1 Gew.-% (bezogen auf eingesetzten Ester). (Vgl. Ullmanns Encyklopädie der techn. Chemie, 4. Auflage, Bd. 15, pg. 256ff, Verlag Chemie, 1978).

Genannt seien z. B. der Hydrochinonmonomethylether und das Hydrochinon.

Die Verbindungen der Formel I sind Flüssigkeiten. Sie eignen sich insbesondere als Vernetzungsreagentien bei der Polymerisation insbesondere von Acrylat- und Methacrylat-Monomeren. Hervorzuheben ist, daß sie gleichzeitig Reglereigenschaften besitzen.

Die folgenden Beispiele dienen zur Erläuterung der Erfindung. Zur Reinheitskontrolle wird die Gaschromatographie herangezogen.

Vorzugsweise erfolgt die Umesterung mit einem Überschuß an den niederen Estern der Acryl- bzw. der Methacrylsäure der Formel III, beispielsweise dem 2 bis 7-fachen Überschuß. Bevorzugt werden Ester der Titansäure wie das käufliche Isopropyltitanat $Ti[OCH(CH_3)_2]_4$ als Umesterungskatalysatoren verwendet, etwa im Bereich von 1/200 bis 1/50 mol, bezogen auf den Alkohol. Bei Verwendung eines Esterüberschusses kann auf Anwendung eines Lösungsmittels verzichtet werden, es können aber auch inerte Lösungsmittel wie Toluol u.ä. verwendet werden. Zweckmäßig wird unter Erwärmen beispielsweise auf Siedetemperatur umgeestert, wobei die Umsetzung durch Entfernung des gebildeten Methanols im azeotropen Gemisch mit beispielsweise Methylmethacrylat gefördert wird.

**Beispiel 1**

Herstellung des 4-Thia-heptandiol-2,6-dimethacrylats

In einem 4-l-Dreihalsrundkolben, ausgestattet mit Thermometer, Rückflußkühler mit Destillationsaufsatz und Rührer, werden 300 g (2 Mol) Thiodipropylenglykol, 1000 g Methylmethacrylat (10 Mol), 0,1 g p-Methoxyphenol und 6,5 g Isopropyltitanat unter Lufteinleiten zum Sieden erhitzt und solange ein Methanol/Methylmethacrylat-Azeotrop abgezogen, bis der Umsatz des Alkohols vollständig ist (6 Std.). Nach Hydrolyse des Katalysators wird das Reaktionsgemisch filtriert und das überschüssige Methylmethacrylat destillativ entfernt. Man erhält das 4-Thia-heptandiol-2,6-dimethacrylat in 85,7 % Ausbeute als gelbe, leicht viskose Flüssigkeit (APHA: 107)

$n_D^{20} = 1{,}4813$ $d^{20}_4 = 1{,}0516$

Reinheit (GC): 95,3 % Diester

**Beispiel 2**

Herstellung des 4-Thio-heptandiol-2,6-diacrylats

Analog Beispiel 1 wird auch das 4-Thiaheptandiol-2.6-diacrylat hergestellt, indem anstelle von Methylmethacrylat die entsprechende Menge Ethylacrylat eingesetzt wird. Reaktionszeit: 6 Std.

Man erhält die Titelverbindung als gelbe, leicht viskose Flüssigkeit. Reinheit (GC): > 95 % Diester.

$n_D^{20} = 1{,}4844$ $d^{20}_4 = 1{,}0811$

## Patentansprüche

1. Ungesättigte Diester von Thia-Alkandiolen der Formel I

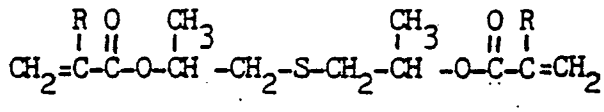

I

worin R für Wasserstoff oder Methyl steht.

2. Verwendung der ungesättigten Diester von Thia-Alkandiolen der Formel I gemäß Anspruch 1 als Monomere in der radikalischen Polymerisation.

## Claims

1. Unsaturated diesters of thia-alkanediols of formula I

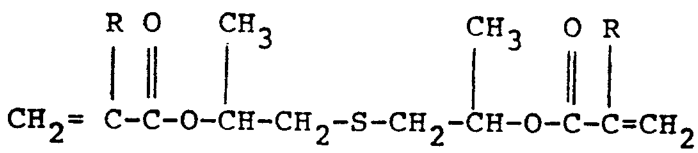

I

wherein R represents hydrogen or methyl.

2. Use of unsaturated diesters of thia-alkanediols of formula I as claimed in claim 1 as monomers in radical polymerization.

## Revendications

1. Diesters non saturés de thia-alcanediols de formule I

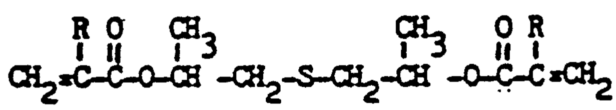

I

dans laquelle R représente un atome d'hydrogène ou un groupe méthyle.

2. Utilisation des diesters non saturés de thia-alcanediols de la formule I selon la revendicatien 1 comme monomères dans la polymérisatien radicalaire.